# EUROPÄISCHE PATENTSCHRIFT

(11) **EP 1 342 487 B1**
(45) Veröffentlichungstag und Bekanntmachung des Hinweises auf die Patenterteilung: **26.07.2006**
(21) Anmeldenummer: 03004917.5
(22) Anmeldetag: 06.03.2003
(51) Int. Cl.: A61N 5/06

(54) **Laserapplikator für die Behandlung von biologischem Gewebe**
Laser applicator for treatment of biological tissue
Applicateur de laser pour le traitement de tissu biologique

(30) Priorität: 07.03.2002 DE 10210007
(43) Veröffentlichungstag der Anmeldung: 10.09.2003
(73) Patentinhaber: Dornier MedTech Laser GmbH, 82234 Wessling (DE)
(72) Erfinder: Illich, Wolfgang, 82234 Wessling (DE); Müller, Thomas, 81377 München (DE)
(74) Vertreter: Grünecker, Kinkeldey, Stockmair & Schwanhäusser Anwaltssozietät

(56) Entgegenhaltungen:
- WO-A-93/25155
- DE-A- 19 739 456
- US-A- 5 367 588

## Beschreibung

Die vorliegende Erfindung betrifft einen Laserapplikator für die Behandlung biologischer Gewebe mit Laserstrahlung, umfassend einen Lichtleiter mit einem die Laserstrahlung führenden Faserkern, wobei der Lichtleiter in einem im Bereich seines distalen Endes liegenden Streubereich Streumittel zum Streuen der Laserstrahlen aus dem Faserkern heraus aufweist.

Ein derartiger Laserapplikator wird zu medizinischen Zwecken beispielsweise in der laserinduzierten interstitiellen Thermotherapie (LITT) und in der photodynamischen Therapie (PDT) eingesetzt. Beim LITT-Verfahren soll das zu behandelnde biologische Gewebe, beispielsweise ein benigner oder maligner Tumor, mit Hilfe von Laserstrahlung beispielsweise auf eine Temperatur zwischen ca. 60°C und 200°C erwärmt werden, damit die Tumorzellen absterben und der Tumor durch Koagulation (Verkochung) zerstört wird. Beim PDT-Verfahren hingegen wird zunächst eine lichtempfindliche Substanz, ein sogenannter Photosensibilitator, im Tumor angereichert. Anschließendes Bestrahlen mit Laserstrahlung bewirkt photochemische Reaktionen, die nach mehreren Schritten letztlich zur Vergiftung und somit Zerstörung des bestrahlten Gewebes führen. Eine thermische Aufheizung des zu behandelnden Gewebes ist hierbei nicht erwünscht.

Bei beiden Methoden bedient man sich in entsprechend ausgestatteten Arztpraxen bzw. Krankenhäusern eines medizinischen Lasergeräts, wie es beispielsweise von der Anmelderin unter der Bezeichnung "Dornier MediLas" angeboten wird, sowie eines geeigneten Laserapplikators, der im wesentlichen einen Lichtleiter mit einem Faserkern umfaßt. Dieser Lichtleiter wird in den Körper des Patienten eingeführt und sein distales Ende, d.h. das vom Lasergerät entfernte Ende, wird in das zu behandelnde biologische Gewebe eingestochen, wobei dieser gesamte Ablauf durch geeignete Methoden, beispielsweise Endoskopie, Ultraschall, Kernspin-Tomographie, überwacht wird.

Auf diese Weise kann die vom Lasergerät erzeugte Laserstrahlung durch den Faserkern bis in den Tumor geleitet werden. Allerdings darf man die Laserstrahlung nicht einfach am distalen Ende aus dem Faserkern austreten lassen, da bei einer typischen Laserleistung von 50 W und einem typischen 5 Faserkerndurchmesser von 600 um am distalen Ende eine derart hohe Leistungsdichte auftreten würde, dass das zu behandelnde Gewebe punktförmig verbrennen, ansonsten aber unbeeinträchtigt bleiben würde.

Daher ist es bereits bei Laserapplikatoren des Stands der Technik bekannt, Streumittel vorzusehen, die bewirken, dass die Laserstrahlung nicht punktförmig am distalen Ende des Lichtleiters austritt, sondern vielmehr entlang eines Bereichs von typischerweise einigen Zentimetern seitlich aus dem Lichtleiter austritt. Hierzu weist der Lichtleiter innerhalb eines Streubereichs, der üblicherweise etwa die letzten zwei bis vier Zentimeter vor seinem distalen Ende umfasst, Streumittel auf, die die Laserstrahlung seitlich aus dem Faserkern heraus streuen.

Aus der DE 19739456 A1 ist eine Applikationsvorrichtung bekannt, die den Oberbegriff des Anspruchs 1 offenbart.

Ein weiterer Laserapplikator ist aus der DE 198 03 460 C1 der Anmelderin bekannt. Bei diesem Laserapplikator ist der Lichtleiter nahezu über seine gesamte Länge von einer Mantelschicht (Cladding) umgeben, im Bereich des Streubereichs jedoch von dieser Mantelschicht wenigstens teilweise befreit. Der Faserkern ist im Streubereich von einem Koppelmedium umgeben, welches den gleichen oder zumindest einen ähnlichen Brechungsindex aufweist wie der Faserkern selbst, so dass die im Faserkern bis in den Streubereich geführte Laserstrahlung zunächst seitlich aus dem Faserkern in das Koppelmedium eintreten kann, ohne hierbei einen nennenswerten Brechungsindexunterschied zu erfahren. In das Koppelmedium, welches beispielsweise durch einen optisch transparenten, aushärtbaren Kleber gebildet sein kann, ist ein Pulver aus einer lichtstreuenden Substanz eingebettet, beispielsweise ein Silikatkeramikpulver. Das zunächst vom Faserkern ungestört in das Koppelmedium eintretende Laserlicht wird von diesen lichtstreuenden Partikeln derart gestreut, dass ein großer Teil des Lichts die äußere Grenzfläche zwischen dem Koppelmedium und der umgebenden Luft unter einem Winkel erreicht, der größer als der geltende kritische Winkel der Totalreflektion ist, und daher aus dem Koppelmedium herausgebrochen wird.

Ein derartiger Laserapplikator wird von der Anmelderin unter der Bezeichnung "Dornier Diffuser-Tip" gewerblich angeboten. Je nach Typ ist der Streubereich bei diesen Laserapplikatoren durch entsprechende Wahl der Abmessungen des Kopplungsmediums unterschiedlich lang gewählt und beträgt zwischen ca. 2 und 4 cm; in diesem Streubereich wird Laserstrahlung nahezu homogen radial vom Faserkern nach außen abgestrahlt.

Auch wenn diese Laserapplikatoren in der Praxis sehr erfolgreich eingesetzt werden, weisen sie doch einige Nachteile auf: Einerseits ist das Herstellungsverfahren insofern kompliziert als bei dem zunächst hergestellten Lichtleiter der Faserkern in seinem späteren Streubereich zunächst von der Mantelschicht befreit werden muß, beispielsweise durch Abschaben oder Abschälen, und dann in diesem Bereich mit dem Koppelmedium überzogen werden muß.

Ein weiterer Nachteil ist darin zu sehen, daß die meisten Koppelmedien nur geringere Temperaturen aushalten als der Faserkern selbst, der in der Regel aus Quarzglas gebildet ist, was eine unerwünschte Leistungsbegrenzung seitens des eingesetzten Lasergeräts erforderlich macht.

Darüber hinaus beeinträchtigt das aufgebrachte Koppelmedium in diesem Bereich die Grundelastizität des Lichtleiters, die für ein problemloses Einführen des Laserapplikators in den Körper des Patienten wichtig ist.

Es ist daher Aufgabe der vorliegenden Erfindung, einen Laserapplikator der eingangs genannten Art vorzuschlagen, der ohne die Verwendung eines derartigen Koppelmediums ein Herausstreuen der Laserstrahlung aus dem Faserkern in Höhe des Streubereichs ermöglicht.

Erfindungsgemäß wird diese Aufgabe bei einem Laserapplikator der eingangs erwähnten Art dadurch gelöst, daß die Streumittel Störbereiche innerhalb des Faserkerns umfassen, deren Brechungsindex von jenem der umgebenden Faserkernbereiche abweicht.

Sobald die vom Lasergerät stammende Laserstrahlung, die sich innerhalb des Faserkerns in Richtung seines distalen Endes ausbreitet und den Faserkern aufgrund der bekannten Totalreflektionseffekte nicht verlassen kann, in den Streubereich gelangt, trifft sie dort auf die Störbereiche, deren Brechungsindex von jenem der die Störbereiche umgebenden Faserbereiche abweicht. Im einfachsten Fall kann es sich bei diesen Störbereichen um mechanische Defekte bzw. Mikrorisse handeln, die z.B. durch mechanische Einwirkung auf den Faserkern in diesem erzeugt werden. An der Grenzfläche zwischen einem Störbereich und dem umgebenden Faserkern ändert sich die Ausbreitungsrichtung der Laserstrahlung schlagartig, wobei je nach Größe des Störbereichs diffuse Streuung oder Reflektion bzw. Transmission an der Grenzfläche als Ursache hierfür anzusehen ist. In beiden Fällen jedoch wird die bisherige Ausbreitung des Laserstrahls im Faserkern, die relativ zur Außenfläche des Faserkerns stets unter einem kleineren Winkel als dem kritischen Winkel der Totalreflektion erfolgte, nachhaltig gestört, und die in Wechselwirkung mit dem Störbereich getretene Laserstrahlung kann unter einem größeren Winkel auf die Außenfläche des Faserkerns treffen und somit herausgebrochen werden. Hierbei kann sogar die Mantelfläche auf Höhe eines Streubereichs belassen werden, man erspart sich also gegenüber Laserapplikatoren des Stands der Technik das aufwendige Abschaben bzw. Abschälen.

Da kein Koppelmedium mit eingelagerten Lichtstreusubstanzen mehr benötigt wird, bleiben die vorteilhafte Hitzebeständigkeit und Elastizität des Lichtleiters erhalten.

Zur Erzeugung der erfindungsgemäß vorgesehenen Störbereiche im Faserkern sind verschiedene Vorgehensweisen möglich: Grundsätzlich kann man die Störbereiche innerhalb des Faserkerns mindestens teilweise durch Bestrahlen mit Teilchenstrahlung erzeugen, beispielsweise mit entsprechend intensiven Elektronen-, Protonen- oder Neutronenstrahlen.

Vorzugsweise ist jedoch vorgesehen, daß die Störbereiche innerhalb des Faserkerns wenigstens teilweise durch Bestrahlen mit Laserstrahlung erzeugt werden. Die Möglichkeit, im Inneren von Glaskörpern mit Hilfe von Laserstrahlen ein Muster zu erzeugen, ohne die Oberfläche des Glaskörpers zu verletzen, ist im Stand der Technik an sich bekannt. Diese "In-Glas-Markierung" wird beispielsweise dazu eingesetzt, im Inneren eines Glasblocks ein dreidimensionales Abbild einer Blume, eines Tiers etc. mit Hilfe fokussierter Laserstrahlung zu erzeugen, um diesen Glasblock als Dekorationsgegenstand zu verwenden. Ebenso ist es bekannt, mit dieser Technik beispielsweise Firmenlogos in Glaskörper "hineinzuschreiben", die als Werbemittel eingesetzt werden sollen. Auf dem Gebiet der Nachrichtentechnik wurde bereits die Möglichkeit beschrieben, im Inneren von Glasfaserkabeln ohne Beeinträchtigung ihrer Oberfläche mit Hilfe dieser Technik Muster in Form von Bragg-Gittern zu erzeugen, vgl. beispielsweise die DE 43 37 103 A1. Unabhängig vom Verwendungszweck des derart bearbeiteten Glaskörpers bzw. Glasfaserkabels basiert diese Technik immer darauf, mit Hilfe von fokussierten Laserblitzen, d.h. kurzen Laserpulsen auf einer Zeitskala von etwa Pikosekunden im Fall einer physikalischen Umwandlung ohne mechanische Störung des Faserkerns bis zu Nanosekunden im Fall der Erzeugung einer mechanischen Schädigung, beispielsweise eines Mikrorisses, lokal im Fokus des Laserblitzes eine derart hohe Leistung im Glas zu konzentrieren, das an der getroffenen Stelle die Struktur des Glases zusammenbricht und ein Störbereich entsteht, dessen Brechungsindex, beispielsweise für sichtbares Licht, von jenem der umgebenden Faserbereiche abweicht.

Die Erzeugung der Störbereiche mit Hilfe der genannten Lasertechnik bietet im Vergleich zur weiter oben erwähnten Bestrahlung des Faserkerns mit Teilchenstrahlung den Vorteil, daß die Größe und die Verteilung der Störbereiche besser kontrolliert werden können. So ist in einer einfach herzustellenden Ausführungsform der Erfindung vorgesehen, daß die Störbereiche im Streubereich des Faserkerns ungeordnet mit im wesentlichen konstanter Dichte verteilt sind. Diese Ausführungsform bietet den Vorteil einer einfachen Herstellung, da beispielsweise der Faserkern ohne besondere Kontrolle mit konstanter Geschwindigkeit durch den Fokus der Laserstrahlen gezogen werden kann, was zu der genannten im wesentlichen konstanten Störbereichsdichte führt. Beim medizinischen Einsatz eines Laserapplikators mit einem derartigen Streubereich im Faserkern wird die aus dem Faserkern herausgestreute Laserstrahlungsintensität vom proximalen Ende des Streubereichs zum distalen Ende hin abnehmen, da sie im wesentlichen proportional zu der im Faserkern verbliebenen, noch nicht herausgestreuten Laserintensität ist, die entsprechend kleiner wird.

Für zahlreiche therapeutische Einsätze ist hingegen eine im wesentlichen konstante Abstrahlung über die gesamte Länge des Streubereichs erwünscht, um beispielsweise einen Tumor, in den der Laserapplikator mit seinem Streubereich hineingedrückt wurde, möglichst gleichmäßig zu bestrahlen. Für derartige Einsätze ist in einer bevorzugten Weiterbildung des erfindungsgemäßen Laserapplikators vorgesehen, daß die Störbereiche mit vom proximalen Ende zum distalen Ende des Streubereichs zunehmender Dichte, insbesondere exponentiell zunehmender Dichte, verteilt sind. Dieses Prinzip ist aus der oben genannten DE 198 03 460 C1 der Anmelderin im Falle eines Faserkerns bereits bekannt, bei dem die von der Mantelschicht befreite Außenfläche pro Längeneinheit in distaler Richtung exponentiell zunimmt, um eine im Streubereich konstante Abstrahlung nach außen zu erreichen.

Zusätzlich zur Dichte der erzeugten Störbereiche, die bei ihrer Erzeugung mit Hilfe fokussierter Laserstrahlung einfach zu kontrollieren ist, bietet diese Technik auch die Möglichkeit, die Größe und den gegenseitigen Abstand der Störbereiche nahezu beliebig einzustellen. Somit kann in einer Weiterbildung des erfindungsgemäßen Laserapplikators dafür gesorgt werden, daß die Größe und der Abstand wenigstens einiger Störbereiche wesentlich größer als die Wellenlänge der Laserstrahlung sind. In diesem Fall wird die Wechselwirkung zwischen der sich im Faserkern ausbreitenden Laserstrahlung und den Störbereichen im Streubereich im wesentlichen durch Reflektion und Brechung an den Grenzflächen zwischen Störbereichen und umgebenden Faserkernbereichen definiert, was zur Herstellung eines Lichtleiters genutzt werden kann, bei dem das aus dem Faserkern herausgestreute Laserstrahlungsfeld eine gewisse Vorwärtscharakteristik aufweist.

Alternativ oder zusätzlich kann jedoch auch vorgesehen sein, daß die Größe und der Abstand wenigstens einiger Störbereiche im Bereich der Wellenlänge der Laserstrahlung sind. In diesem Fall dominiert Streuung als maßgebliche Wechselwirkung zwischen der sich im Faserkern ausbreitenden Laserstrahlung und den Störbereichen, was bei ausreichend hoher Störbereichsdichte zu einem ähnlich homogenen Abstrahlungsfeld führt, wie beim eingangs bereits geschilderten "Dornier Diffuser-Tip".

Für spezielle Anwendungen sind bei dem erfindungsgemäßen Laserapplikator wenigstens einige Störbereiche in Gestalt eines Beugungsgitters angeordnet. Die Möglichkeit, derartige Beugungsgitter, insbesondere Bragg-Gitter in Glasfaserkabeln zu erzeugen, ist in der oben genannten DE 43 37 103 A1 detailliert beschrieben. Der erfindungsgemäße Laserapplikator sendet Laserstrahlung nur unter bestimmten Winkeln gegenüber einem solchen Bragg-Gitter und somit auch gegenüber der Längsachse des Lichtleiters aus, entsprechend der ringförmigen zirkumferentiellen Strahlung der im Stand der Technik bekannten "Dornier ITT-Lichtleiter".

Bei allen genannten Ausführungsformen kann zweckmäßigerweise vorgesehen sein, daß der Faserkern im Bereich seines distalen Endes von einer transparenten Hülse umgeben ist. Eine derartige Hülse, die beispielsweise aus Glas oder aus einem flexiblen Teflonschlauch gebildet sein kann, gewährleistet einen mechanischen Schutz des Faserkerns.

Figuren 1-3 zeigen Laserapplikatoren, die aus Dokument DE 197 39 456 bekannt sind.
- Fig.1: ein Laserapplikator mit verhältnismäßig großen Störbereichen;
- Fig. 2: ein Laserapplikator mit im wesentlich homogen verteilten kleinen Störbereichen; und
- Fig. 3: ein Laserapplikator, bei der die Störbereichsdichte vom proximalen zum distalen Ende des Streubereichs zunimmt.

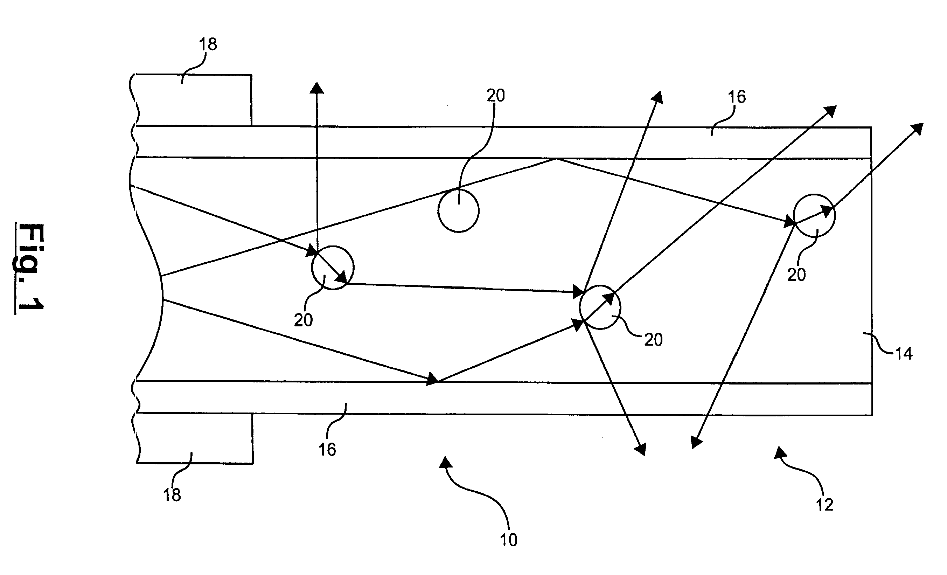

Fig. 1 zeigt einen Querschnitt durch einen Laserapplikator 10 in Höhe eines seiner Streubereiche. Üblicherweise weist ein derartiger Laserapplikator 10 nur einen einzigen Streubereich auf, nämlich an seinem distalen Ende, d.h. an dem in Fig. 1 rechten Ende, das von einem in Fig. 1 nicht gezeigten Lasergerät am linken Ende des Laserapplikators 10 entfernt ist. Der Laserapplikator 10 umfaßt einen Lichtleiter 12, der aus einem zentralen Faserkern 14 und einer um den Faserkern 14 herum angeordneten Mantelschicht (Cladding) 16 gebildet ist. Der Lichtleiter 12 ist über den Großteil seiner Länge, die bis zu mehreren Metern betragen kann, von einer Umhüllung 18 umgeben, die ihn schützt. Das distale Ende dieser Umhüllung 18 ist in Fig. 1 links zu erkennen.

Die vom nicht gezeigten Lasergerät in den Faserkern 14 eingespeiste Laserstrahlung wird im Faserkern 14 bekanntlich aufgrund der Totalreflektion an der Grenzfläche zwischen dem Faserkern 14 und der ihn umgebenden Mantelschicht 16 "eingeschlossen". Während daher Laserstrahlen, die sich gegenüber dieser Grenzfläche unter einem größeren Winkel als dem kritischen Winkel der Totalreflektion ausbreiten, bereits in der Nähe des proximalen Endes des Laserapplikators 10 aus dem Faserkern herausgebrochen wurden, sind im Bereich seines in Fig. 1 gezeigten distalen Endes nur noch solche Laserstrahlen im Faserkern 14 vorhanden, die sich in ihm verhältnismäßig "flach" ausbreiten, d.h. relativ zur genannten Grenzfläche unter einem kleineren Winkel als dem kritischen Winkel der Totalreflektion. Ohne besondere Streumittel würden diese Laserstrahlen, von denen in Fig. 1 links einige in Form von Pfeilen eingezeichnet sind, den Faserkern 14 an seinem in Fig. 1 rechten Ende verlassen, was zu den eingangs geschilderten Problemen einer zu großen Leistungsdichte führen würde, die keine zweckmäßige Behandlung des biologischen Gewebes erlaubt, beispielsweise keine erfolgreiche Koagulation eines zu beseitigenden Tumors.

Daher weist der Faserkern 14 in seinem in Fig. 1 gezeigten Streubereich mehrere Störbereiche 20 auf, deren Größe und gegenseitiger Abstand wesentlich größer als die Wellenlänge der eingesetzten Laserstrahlung ist, d.h. in der Regel wesentlich größer als ca. 1 µm. Diese Störbereiche 20 sind im Faserkern 14 durch Bestrahlen mit intensiven fokussierten Laserlichtpulsen erzeugt worden, die dazu führen, daß die Struktur des Faserkernmaterials im Fokus geändert wird und somit ein Störbereich 20 entsteht, dessen Brechungsindex von jenem des umgebenden Faserkerns 14 abweicht.

Trifft nun ein Laserstrahl, der sich im Faserkern 14 von seinem in Fig. 1 linken proximalen Ende zu seinem rechten distalen Ende ausbreitet, auf einen solchen Störbereich 20 mit abweichendem Brechungsindex, so treten an der Grenzfläche zwischen dem umgebenden Faserkernbereich und dem Störbereich 20 die bekannten Richtungsänderungen durch Reflektion und Brechung auf, die i.w. gemäß den Snellius-Gesetzen vom Verhältnis der beiden betrachteten Brechungsindizes abhängen.

Wie man an einigen der in Fig. 1 eingezeichneten Laserstrahlen erkennt, führen beide Phänomene, nämlich sowohl Reflektion als auch Transmission unter Brechung, zum Auftreten von Laserstrahlen, die nicht mehr unter einem hinreichend flachen Winkel auf die Grenzfläche zwischen dem Faserkern 14 und der Mantelschicht 16 treffen, sondern vielmehr unter einem Winkel oberhalb des kritischen Winkels der Totalreflektion. Diese Strahlen können somit zunächst in die Mantelschicht 16 transmittiert werden, und bei geeignetem Verhältnis zwischen den Brechungsindizes der Mantelschicht 16 und der umgebenden Luft auch vollständig aus dem Lichtleiter 12 heraus nach außen. Selbstverständlich kann die Mantelschicht 16 auf Höhe des gezeigten Streubereichs auch entfernt worden sein, bzw. der Lichtleiter 12 zumindest in diesem Streubereich von Anfang an ohne Mantelschicht 16 hergestellt worden sein. In diesem Fall ist der kritische Winkel der Totalreflektion an der Außenfläche des Faserkerns 14 nur durch den Brechungsindex des Faserkerns 14 und jenen der umgebenden Luft bestimmt.

In Fig. 1 ist aus Gründen der Übersichtlichkeit eine äußerst geringe Dichte an Störbereichen 20 innerhalb des dargestellten Streubereichs eingezeichnet. Es versteht sich jedoch, daß man in der Praxis derartige Störbereiche 20 mit wesentlich größerer Dichte im Streubereich des Faserkerns 14 erzeugen wird, um die sich im Faserkern 14 ausbreitenden Laserstrahlen vor Erreichen des distalen Endes des Lichtleiters 12 möglichst vollständig aus dem Lichtleiter 12 herauszustreuen.

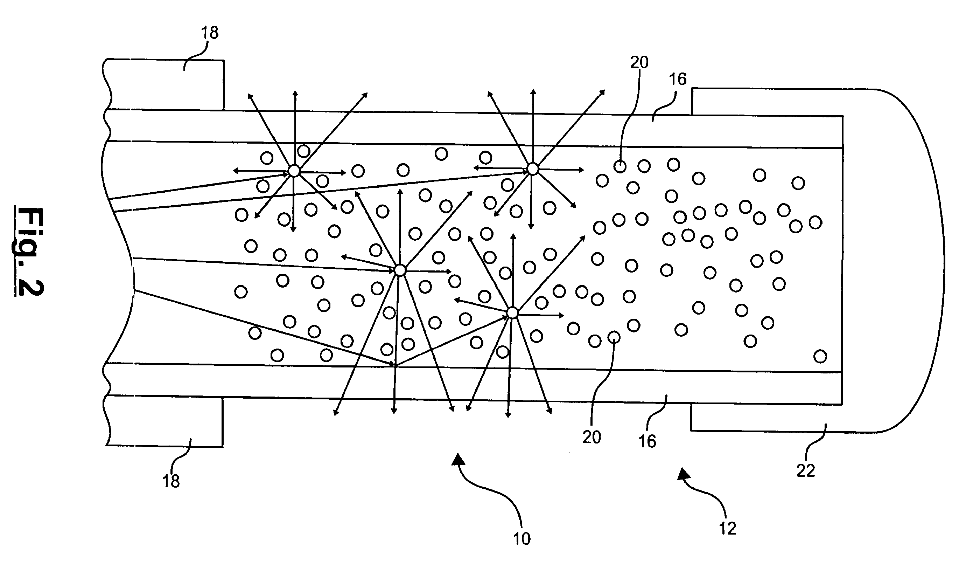

Fig. 2 zeigt ein Laserapplikator 10, bei der identische bzw. ähnliche Elemente die gleichen Bezugszeichen tragen wie in Fig. 1.

In der schematischen Darstellung von Fig. 2 sind die Störbereiche 20 derart dimensioniert, daß ihre Größe und ihr Abstand im Bereich der Wellenlänge der Laserstrahlung liegen, d.h. beispielsweise im Bereich von 1 µm. Es ist daher darauf hinzuweisen, daß die Abmessungen in Fig. 2 nicht maßstabsgerecht sind, da beispielsweise ein typischer Durchmesser des Faserkerns 14 bei ca. 600 µm liegt.

Im Gegensatz zur Variante von Fig. 1 ist in Fig. 2 aufgrund der dortigen Abmessungen und Abstände der Störbereiche 20 Streuung der dominierende Wechselwirkungsmechanismus zwischen der sich im Faserkern 14 ausbreitenden Laserstrahlung und den Störbereichen 20. Dies ist in Fig. 2 durch Pfeile an einigen Störbereichen 20 vereinfacht dargestellt, die von Laserstrahlen im Faserkern 14 getroffen werden und das Laserlicht im wesentlichen homogen in einen Raumwinkel von 4 Pi streuen.

In der Variante gemäß Fig. 2 sind die Störbereiche 20 im dargestellten Streubereich des Laserapplikators 10 weitgehend ungeordnet und mit im wesentlichen konstanter Dichte verteilt. Daher wird der Großteil der Laserstrahlung bereits im proximalen Bereich des Streubereichs heraus gestreut werden, wohingegen nur eine sehr geringe Rest-Laserstrahlung innerhalb des Faserkerns 14 das in Fig. 2 rechte distale Ende des Faserkerns 14 erreicht und dort aus dem Lichtleiter 12 heraus gestreut wird. Somit liefert der Laserapplikator 10 gemäß der Variante der Fig. 2 eine vom proximalen zum distalen Ende des dargestellten Streubereichs abnehmende Streuintensität. Wie genauere Untersuchungen zeigen, erfolgt diese Abnahme exponentiell. Diese Variante des Laserapplikators 10 ist besonders einfach herzustellen, da keinerlei Ordnung der Störbereiche 20 einzustellen ist, und eignet sich beispielsweise bei der Behandlung von Tumoren, die an der Oberfläche stärker als im Inneren durch das seitlich heraus gestreute Laserlicht erwärmt werden sollen.

Man erkennt in Fig. 2 rechts ferner eine transparente Hülse 22 auf dem distalen Ende des Lichtleiters 12, die diesen insbesondere beim Einführen in den Körper des Patienten, vor allem in das zu behandelnde biologische Gewebe, schützt.

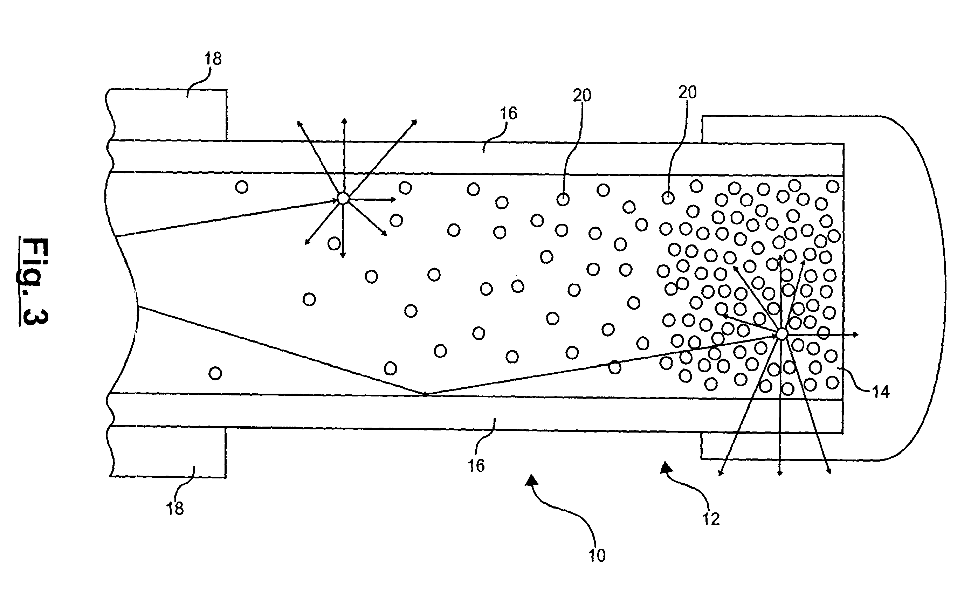

Fig. 3 zeigt eine weitere Variante des Laserapplikators 10, bei der eine entlang des gesamten Streubereichs im wesentlichen homogene Abstrahlintensität erreicht wird. Zu diesem Zweck sind die Störbereiche 20 im Gegensatz zur Variante gemäß Fig. 2 nicht homogen im Streubereich des Faserkerns 14 verteilt, sondern vielmehr mit einer vom in Fig. 3 linken proximalen Ende zum rechten distalen Ende zunehmenden Dichte. An dem in Fig. 3 linken proximalen Ende des gezeigten Streubereichs trifft zwar eine sehr hohe Laserstrahlungsintensität ein, es sind dort jedoch nur wenige Streuzentren in Form von Störbereichen 20 vorhanden, die Laserstrahlung aus dem Lichtleiter 12 heraus streuen können. Andererseits kommt an dem in Fig. 3 rechten distalen Ende des Faserkerns 14 nur noch eine sehr geringe Laserstrahlungsintensität an, da bereits ein beträchtlicher Teil der Laserstrahlung aus dem Lichtleiter 12 herausgestreut worden ist. Diese geringe verbliebene Laserstrahlungsintensität trifft dort jedoch auf die maximale Störbereichsdichte, so daß insgesamt eine im wesentlichen ebenso große Streuintensität erreicht wird wie am proximalen Ende des Streubereichs. Insbesondere ergibt sich bei einer exponentiellen Zunahme der Streubereichsdichte in Fig. 3 von links nach rechts eine im wesentlichen homogene Intensitätsverteilung des radial aus dem Lichtleiter 12 heraus gestreuten Laserstreulichts.

Ein erfindungsgemäßer Laserapplikator 10 könnte weitere Merkmale aufweisen, die bei Lichtleitern des Stands der Technik bekannt sind. Beispielsweise könnte ein erfindungsgemäßer Laserapplikator 10 eine Düsenspitze und einen Spülschlauch aufweisen, damit ein gasförmiges oder flüssiges Spülmedium zum Schutz des distalen Faserkernendes, zum Freispülen der Blutungsstelle, zur Water-Jet-Koagulation und zur endoskopischen Sichtverbesserung herangeführt werden kann. Grundsätzlich ist es sogar vorstellbar, mehrere Streubereiche hintereinander in einem Laserapplikator 10 vorzusehen, an denen jeweils ein Teil der im Faserkern 14 geführten Strahlen heraus gestreut wird. Ferner versteht es sich, daß die Störbereiche 20 auch andere Verteilungen aufweisen können, als sie in den Fig. 1 bis 3 dargestellt sind, und daß insbesondere die Gestalt eines einzelnen Störbereichs 20 keineswegs auf die in den Fig. vereinfacht gezeigte "Kugelform" beschränkt ist, sondern vielmehr aufgrund der oben beschriebenen "In-Glas-Markierungstechnik" nahezu beliebig geformte Störbereiche 20 in den Faserkern 14 "hineingeschrieben" werden können. Ebenso versteht es sich, daß die Frage der Größe und des gegenseitigen Abstands der Störbereiche 20 weitgehend unabhängig ist von der Frage, wie der Verlauf der Störbereichsdichte vom proximalen zum distalen Ende des Streubereichs eingestellt wird. Daher kann selbstverständlich auch bei dem in Fig. 1 gezeigten Beispiel verhältnismäßig größer Störbereiche 20 ein entlang der Lichtausbreitungsrichtung exponentieller Anstieg der Dichte dieser Störbereiche 20 eingestellt werden.

## Patentansprüche

1. Laserapplikator (10) für die Behandlung biologischer Gewebe mit Laserstrahlung, umfassend einen Lichtleiter (12) mit einem die Laserstrahlung führenden Faserkern (14), wobei der Lichtleiter (12) in einem im Bereich seines distalen Endes liegenden Streubereich Streumittel zum Streuen der Laserstrahlen aus dem Faserkern (14) heraus aufweist,
wobei die Streumittel Störbereiche (20) innerhalb des Faserkerns (14) umfassen, deren Brechungsindex von jenem der umgebenden Faserkernbereiche abweicht,
**dadurch gekennzeichnet,**
**dass** wenigstens einige Störbereiche (20) in Gestalt eines Beugungsgitters angeordnet sind.

2. Laserapplikator (10) nach Anspruch 1, **dadurch gekennzeichnet, dass** die Störbereiche (20) innerhalb des Faserkerns (14) wenigstens teilweise durch Bestrahlen mit Teilchenstrahlung erzeugt sind.

3. Laserapplikator (10) nach Anspruch 1 oder 2, **dadurch gekennzeichnet, dass** die Störbereiche (20) innerhalb des Faserkerns (14) wenigstens teilweise durch Bestrahlen mit Laserstrahlung erzeugt sind.

4. Laserapplikator (10) nach einem der vorhergehenden Ansprüche, **dadurch gekennzeichnet, dass** die Störbereiche (20) im Streubereich des Faserkerns (14) ungeordnet mit im wesentlichen konstanter Dichte verteilt sind.

5. Laserapplikator (10) nach einem der Ansprüche 1 bis 3, **dadurch gekennzeichnet, dass** die Störbereiche (20) mit vom proximalen Ende zum distalen Ende des Streubereichs zunehmender Dichte, insbesondere exponentiell zunehmender Dichte, verteilt sind.

6. Lasergerät und Laserapplikator (10) nach einem der vorhergehenden Ansprüche, **dadurch gekennzeichnet, dass** die Größe und der Abstand wenigstens einiger Störbereiche (20) wesentlich größer als die Wellenlänge der Laserstrahlung des zugeordneten Lasergeräts sind.

7. Lasergerät und Laserapplikator (10) nach einem der vorhergehenden Ansprüche, **dadurch gekennzeichnet, dass** die Größe und der Abstand wenigstens einiger Störbereiche (20) im Bereich der Wellenlänge der Laserstrahlung des zugeordneten Lasergeräts sind.

8. Laserapplikator (10) nach einem der vorhergehenden Ansprüche, **dadurch gekennzeichnet, dass** der Faserkern (14) im Bereich seines distalen Endes von einer transparenten Hülse (22) umgeben ist.

## Claims

1. Laser applicator (10) for the treatment of biological tissues with laser radiation, comprising an optical waveguide (12) with a fibre core (14) conducting the laser radiation, said optical waveguide (12) exhibiting, in a scattering region situated in the region of its distal end, scattering means for scattering the laser beams out of the fibre core (14),
said scattering means comprising disturbance regions (20) within the fibre core (14), the refractive index of said disturbance regions differing from that of the surrounding fibre-core regions,
**characterised in that**
at least some disturbance regions (20) are arranged in the form of a diffraction grating.

2. Laser applicator (10) according to Claim 1, **characterised in that** the disturbance regions (20) within the fibre core (14) are generated at least partially by irradiating with particle radiation.

3. Laser applicator (10) according to Claim 1 or 2, **characterised in that** the disturbance regions (20) within the fibre core (14) are generated at least partially by irradiating with laser radiation.

4. Laser applicator (10) according to one of the preceding claims, **characterised in that** the disturbance regions (20) in the scattering region of the fibre core (14) are distributed randomly with substantially constant density.

5. Laser applicator (10) according to one of Claims 1 to 3, **characterised in that** the disturbance regions (20) are distributed with increasing density, in particular with exponentially increasing density, from the proximal end to the distal end of the scattering region.

6. Laser instrument and laser applicator (10) according to one of the preceding claims, **characterised in that** the size and the spacing of at least some disturbance regions (20) are substantially greater than the wavelength of the laser radiation of the assigned laser instrument.

7. Laser instrument and laser applicator (10) according to one of the preceding claims, **characterised in that** the size and the spacing of at least some disturbance regions (20) are within the range of the wavelength of the laser radiation of the assigned laser instrument.

8. Laser applicator (10) according to one of the preceding claims, **characterised in that** the fibre core (14) is surrounded in the region of its distal end by a transparent sleeve (22).

## Revendications

1. Applicateur laser (10) pour le traitement de tissus biologiques par faisceau laser, comprenant un guide d'ondes optiques (12) avec un noyau de fibre (14) guidant le faisceau laser, dans lequel le guide d'ondes optiques (12) présente, dans une zone de dispersion située dans la zone de son extrémité distale, des moyens de dispersion pour disperser le faisceau laser à partir du noyau de fibre (14), dans lequel les moyens de dispersion comprennent à l'intérieur du noyau de fibre (14) des zones de brouillage (20) dont l'indice de réfraction diffère de celui des zones de noyau de fibre environnantes, **caractérisé en ce qu'**au moins quelques-unes des zones de brouillage (20) sont disposées sous la forme d'un réseau de diffraction.

2. Applicateur laser (10) selon la revendication 1, **caractérisé en ce que** les zones de brouillage (20) à l'intérieur du noyau de fibre (14) sont produites au moins en partie par une irradiation avec un rayonnement de particules.

3. Applicateur laser (10) selon la revendication 1. ou 2, **caractérisé en ce que** les zones de brouillage (20) à l'intérieur du noyau de fibre (14) sont produites au moins en partie par irradiation par faisceau laser.

4. Applicateur laser (10) selon l'une des revendications précédentes, **caractérisé en ce que** les zones de brouillage (20) dans la zone de dispersion du noyau de fibre (14) sont réparties de façon aléatoire avec une densité essentiellement constante.

5. Applicateur laser (10) selon l'une des revendications 1 à 3, **caractérisé en ce que** les zones de brouillage (20) sont réparties avec une densité augmentant de l'extrémité proximale à l'extrémité distale de la zone de dispersion, en particulier avec une densité augmentant de façon exponentielle.

6. Appareil laser et applicateur laser (10) selon l'une quelconque des revendications précédentes, **caractérisés en ce que** la grandeur et l'écartement d'au moins quelques zones de brouillage (20) sont sensiblement plus grands que la longueur d'onde du faisceau laser de l'appareil laser associé.

7. Appareil laser et applicateur laser (10) selon l'une quelconque des revendications précédentes, **caractérisés en ce que** la grandeur et l'écartement d'au moins quelques zones de brouillage (20) sont dans la zone de la longueur d'onde du faisceau laser de l'appareil laser associé.

8. Applicateur laser (10) selon l'une quelconque des revendications précédentes, **caractérisé en ce que** le noyau de fibre (14) est entouré, dans la zone de son extrémité distale, par une gaine transparente (22).
